# EUROPEAN PATENT APPLICATION

(11) **EP 1 800 706 A1**
(43) Date of publication of application: **27.06.2007**
(21) Application number: 05078024.6
(22) Date of filing: 22.12.2005
(51) Int. Cl.: A61M 16/04, B29C 49/00

(54) **Laryngeal mask**

(71) Applicant: Unomedical A/S, 3460 Birkerød (DK)
(72) Inventor: Poulsen, Sylvia Ewa Krol, 30020 gen. Meru Valley (MY)
(74) Representative: Elmeros, Claus

(57) **Abstract**

The present invention concerns improvements relating to a laryngeal mask comprising an inflatable cuff (1), a mask element (2) and a tubular element (3), wherein the mask element (2) is provided with a reinforcement tongue (7) for receiving and supporting the tubular element (3) and providing an extensive contact for assembly, e.g. by adhesion, between the mask element (2) and the tubular element (3).

## Description

The present invention relates to improvements of a laryngeal mask.

This type of product is based on the artificial airway device described in US 4,509,514. Laryngeal masks are used to facilitate lung ventilation in an unconscious patient, e.g. during anaesthesia, and more specifically the masks are designed in such a way that they may be placed in the oropharynx of the patient in order to prevent airway obstruction.

The masks comprise an airway tube, a mount and an inflatable mask or cuff at one end. The tube is inserted into the patient's mouth so that this cuff is located in the hypopharynx and so that the mask forms a seal in this region with the surrounding tissue. An air inflation tube is provided for inflating or deflating the cuff. This air flow tube Is provided along the airway tube, as shown e.g. in US 8,705,318.

Other examples of laryngeal masks are known from EP 1 219 316 and WO 2004/089453 as well as WO 00/61213 and WO 2004/064908.

Various drawbacks have been Identified by the known airway devices either in the manufacture or in relation to the use. Consequently, it is an object of the invention to provide improvements in relation to a laryngeal mask and its manufacture.

These objects are achieved by the invention.

Various advantageous aspects of the invention are described in the accompanying claims.

By the invention the insertion is facilitated as the frontal portion of the laryngeal mask, i.e. the frontal portion of the mask portion and the cuff, will not flip backwards with the risk of blocking the air passage way in which the mask is being inserted. This is due to design with the thicker, harder or otherwise more stiff upper side of the cuff and/or the "inner mounting" of the mask portion in the intermediate cuff balloon.

In the following, the invention is described in detail with reference to the drawings, in which:
Fig. 1 is a principle side view of a laryngeal mask according to a first embodiment of the Invention;
figures 2 to 5 show different side views according to embodiments of a connector piece of a laryngeal mask according to the invention;
figures 6 to 8 show front views according to embodiments of a connector piece of a laryngeal mask according to the invention;
fig. 9 is a side view of a laryngeal mask according to another embodiment of the invention;
figures 10 to 12 show cross-section views of fig. 9;
fig. 13 is a principle side view of the cuff manufacturing tool prior to the start of the process;
fig. 14 is a principle top view of the intermediate balloon product in the cuff manufacturing:
fig. 15 shows the same in a side view;
fig. 16 is a principle side view of a further cuff manufacturing step;
fig. 17 is a principle side view of a cuff according to an embodiment of the invention;
fig, 18 is a top view of the cuff shown in fig. 17;
fig. 19 is a principle side view of an assembly with the intermediate cuff balloon with a mask portion mounted therein according to a second embodiment of the invention;
fig. 20 is the finished cuff according to this second embodiment;
fig. 21 is an explanatory illustration of the cuff manufacturing step succeeding the assembly of ftg.19;
fig. 22 is a schematic side view of a laryngeal mask according to a further embodiment of the invention;
fig. 23 is a bottom view of the laryngeal mask of fig. 22; and
fig. 24 is a perspective view of a mask portion of the laryngeal mask of figures 22 and 23.

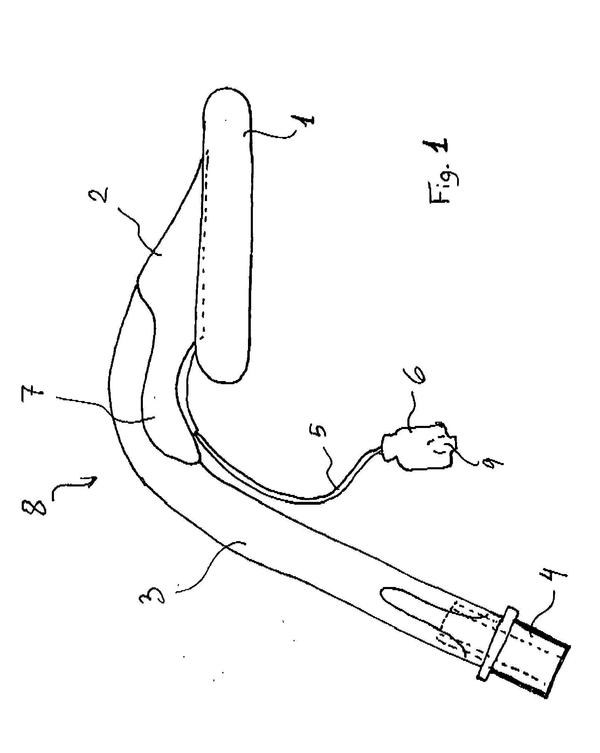

With reference to the figure 1, the laryngeal mask includes a tubular element 3, a mask element 2, where said two elements are joined together by glue or the like. A cuff 1 is fixed to the mask element 2. The cuff 1 is inflatable so that it blocks the relevant areas in the throat. An air inflation tube 5 connects the cuff 1 to a pilot balloon 6 which may be supplied with air from an external air supply source (not shown) via a non-retum valve 9 in the pilot balloon 6, whereby the cuff 1 is inflated and the air pressure in the cuff 1 can be constantly monitored by watching the pilot balloon 6.

The pilot balloon 6 has a volume which is at least three times smaller than the volume of the cuff 1. Hereby, it is possible to control the inflation of the cuff 1 accurately by monitoring the pilot balloon 6.

In the distal end of the tubular element 3, a connector piece 4 Is Inserted into the end of the tubular element 3.

The tubular element 3 is preferably provided with a bend 8 on approx. 90° which facilitates the use of the laryngeal mask. The mask element 2 is adapted to receiving the tubular element 3. On the inside of the bend 8 of the tubular element 3, the mask element 2 is provided with a supporting reinforcement tongue 7. This tongue 7 supports the tubular element 3 in the bend region and reinforces the laryngeal mask in the area of the bend 8 and prevents the tubular element 3 of the laryngeal mask from kinking, i.e. collapsing during insertion. The tongue 7 is provided with a shape corresponding to the outer contour of the tubular element 3. The inside of this reinforcement tongue 7 of the mask element 2 ensures a large area of contact between the tubular element 3 and the mask element 2, which provide a large adhesive contact ensuring a firm adhesive assembly between the tubular element 3 and the mask element 2.

On the inside of the bend 8, a recess may be provided in which an air inflation tube 5 may be provided connecting the inflatable cuff 1 with an air supply source. Alternatively, this air inflation tube 5 could be provided in a recess inside the tube 3. In yet another embodiment, the air inflation tube is connected directly to a flow channel provided in the mask element 2 where this flow channel is in flow communication with the cuff 1.

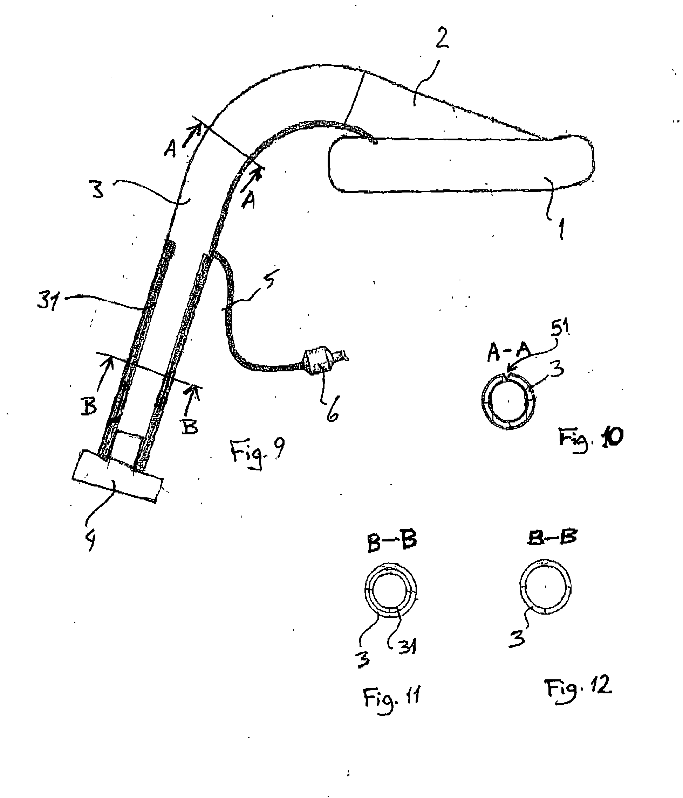

As shown in figure 9, tubular reinforcement 31 may be provided on the tubular element 3. This reinforcement may constitute a so-called biting bloc, which is preferably provided by extruding a tubular piece which is fitted inside the tube 3.

As an alternative or in supplement to the tubular piece 31, the biting bloc may be provided by the connector piece 4, which is provided with a protruding portion 43, which protrudes into the mouth end of the tubular element 3 and is circumscribed by the tube 3. This protrusion 43 is preferably between one to five times the diameter of the tube, more preferably between one and three times the diameter of tube.

With reference to figures 2 to 8, the connector piece 4 is provided at the distal end - i.e. mouth end - of the laryngeal mask. The connector piece 4 is made of a material having a Shore A hardness of 90 plus or minus 20. The tube element 3 and the mask portion 2 are preferably made with a Shore A hardness of 65. This means that the distal end of the tubular element 3 is resilient compared to the insert protrusion of the connector piece 4 and that the distal end may be deformed as the connector piece 4 is inserted.

The Shore hardness is measured with an apparatus known as a Durometer and consequently is also known as 'Durometer hardness'. The hardness value is determined by the penetration of the Durometer indenter foot into the sample. Because of the resilience of rubbers and plastics, the indentation reading my change over time - so the indentation time is sometimes reported along with the hardness number. The ASTM test method designation is ASTM D2240 00 and is generally used in North America. Related methods include ISO 7619 and ISO 868; DIN 53505; and JIS K 6301, which was discontinued and superseded by JIS K 6253.

Laryngeal mask are provided in different sizes according to the size of the patients. Accordingly, the diameter of the tube element 3 is different and consequently the connector piece 4 must also be adapted to the sizes. In the figures 2 to 4, three examples of connector pieces 4 adapted for different tube element diameters are shown.

The connector piece 4 includes an external connector portion 41, a stop flange 42 and an insertion portion 43. A bore 44 is provided through the entire connector piece 4. In a first embodiment of the invention, the bore 44 is provided with a circular cross-section with or without different diameters in the connector portion 41, the flange portion 42 and the insertion portion 43 depending on the size of laryngeal mask. However, it is realised that other cross-section shapes could be provided, e.g. an elliptical shape and/or internal recesses in the bore 44 for accommodating the air inflation tube or the like.

The connector portion 41 is cylindrical having a circular cross-section. The dimensions of the connector portion 41 are the same for all sizes of the laryngeal masks so that it is connectable to standard ventilation equipment, such as a respiratory apparatus or an anaesthetic system, when the laryngeal mask Is placed in a patient irrespective of the size of the laryngeal mask and the patient. The insertion portion 43 is dimensioned according to the size of the tube element 3. The insertion portion 43 is preferably provided with a length L, which is one to five times the Internal diameter of the tube element 3, more preferably one to three times.

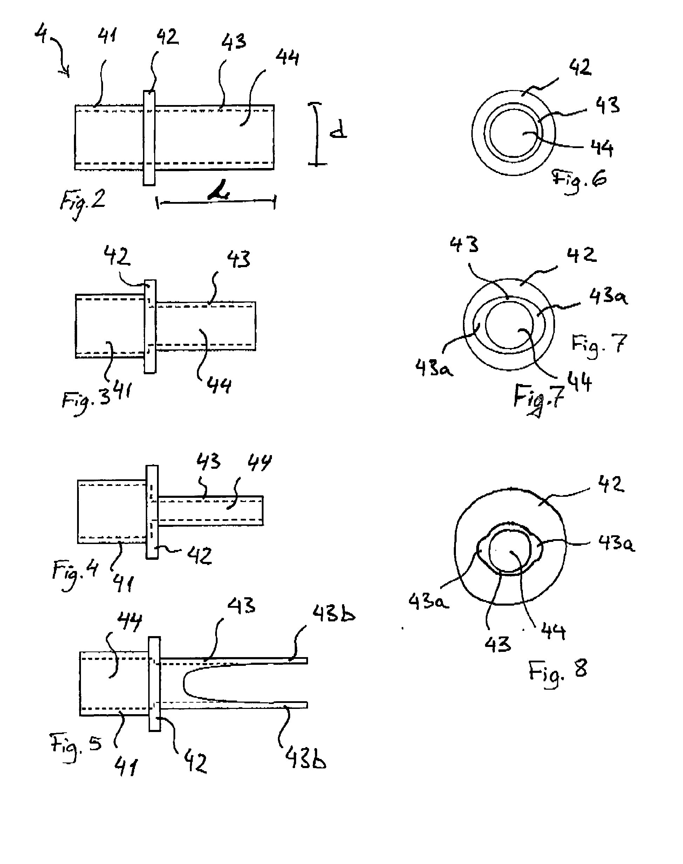

The insertion portion 43 may be provided with a circular cross-section as shown in fig. 6. However, In another embodiment shown in fig, 7 the cross-section is oval or elliptical thereby utilising that the distal end of the tubular element 3 is more resilient than the insert protrusion of the connector piece 4 whereby the distal end may be deformed as the connector piece 4 is Inserted. This is advantageous as the side portions 43a of the insertion portion 43 are reinforced and thereby the tube is prevented from being collapsed by a patient's bite on the mouth end of the laryngeal mask. In fig. 8, yet another embodiment of the insertion portion cross-section is shown with side portions 43a reinforcing the tube and thereby prevents kinking or otherwise collapse of the tube at the mouth end of the laryngeal mask.

The parts of the laryngeal mask, i.e. the tubular element 3, the mask element 2, the inflatable cuff 1 and the connector piece 4 may be produced by extrusion, injection moulding, blow moulding or any combination thereof. The materials used for the four parts are selected in accordance with the desired characteristics of the individual parts.

As an alternative to the mask portion design of figure 1, it is realised that the tubular element 3 and the mask element 2 may be integrally formed as a tubular airway mask element by injection moulding.

The cuff 1 is fixed to the mask element 2. The cuff 1 is inflatable so that it blocks the relevant areas in the throat. The cuff 1 is manufactured by blow moulding, as an extruded tubular piece is heated and blown into a balloon inside a cavity in a blow moulding form. The balloon is cut off from the tubular piece and removed for further processing. This further processing includes providing a slit in the middle portion of the balloon and thereby deflating the balloon and subsequently turning the balloon inside out so that inner side becomes the outer side. The advantage by this process is that the assembly between the upper and lower mould parts now faced inside and the burrs or flashes along this assembly line also face inside and cannot irritate the tissue in the patient's throat. After this, the "reversed" balloon is formed into an annular cuff by stamping out and welding together a central aperture generally corresponding to the footprint of the mask element to which the cuff is to be fixed.

The cuff 1 is preferably with different dimensions. As shown in the top and side views of the cuff 1 the cuff is provided with a wall thickness of 0.2 to 0.6 mm. In a preferred embodiment, the cuff 1 may be provided with a larger thickness of the upper side than the underside of the cuff.

Moreover, the cuff 1 is provided with a reinforcement strip 7 in the front end of the cuff 1 where the thickness of the inflatable cuff wall is approx. 0.8 to 0.9 mm. This reinforcement is provided during the manufacture of the cuff 1 by extruding or otherwise providing a strip parallel to the extruded tubular piece which immerged together with tube tubular piece from the heated extrusion form. The temperature is approx. 170° C and by the subsequent blow moulding process, the strip and the cuff balloon will melt together for providing the reinforced portion of the cuff 1. By providing the reinforcement on the resulting blow-moulded balloon, no sharp edges will occur, as the balloon is reversed.

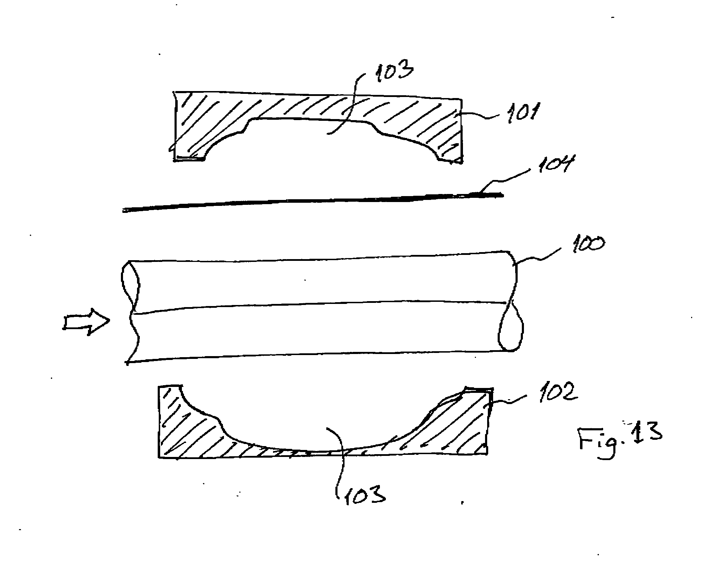

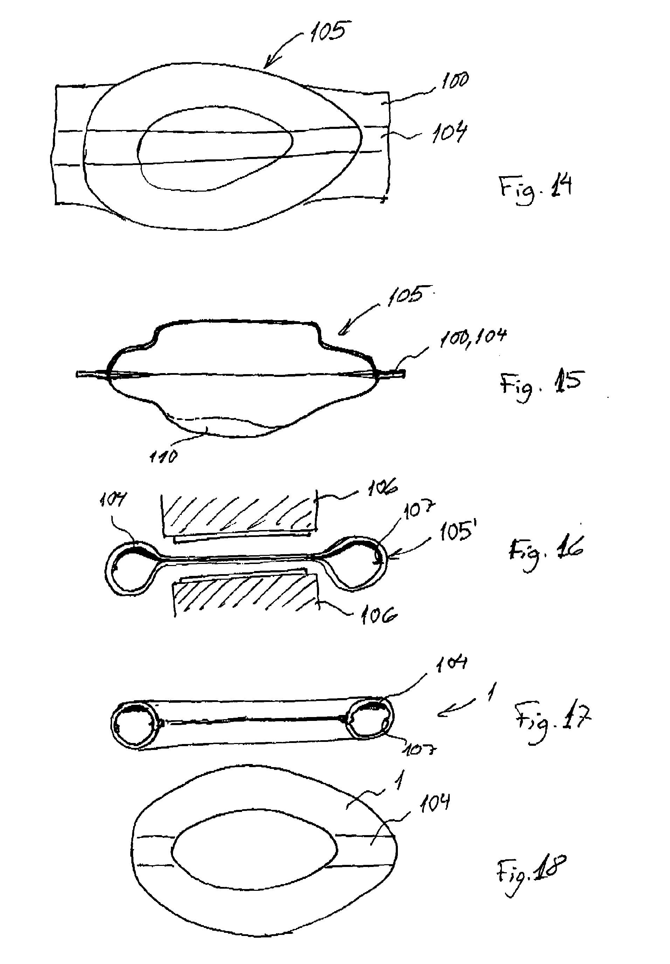

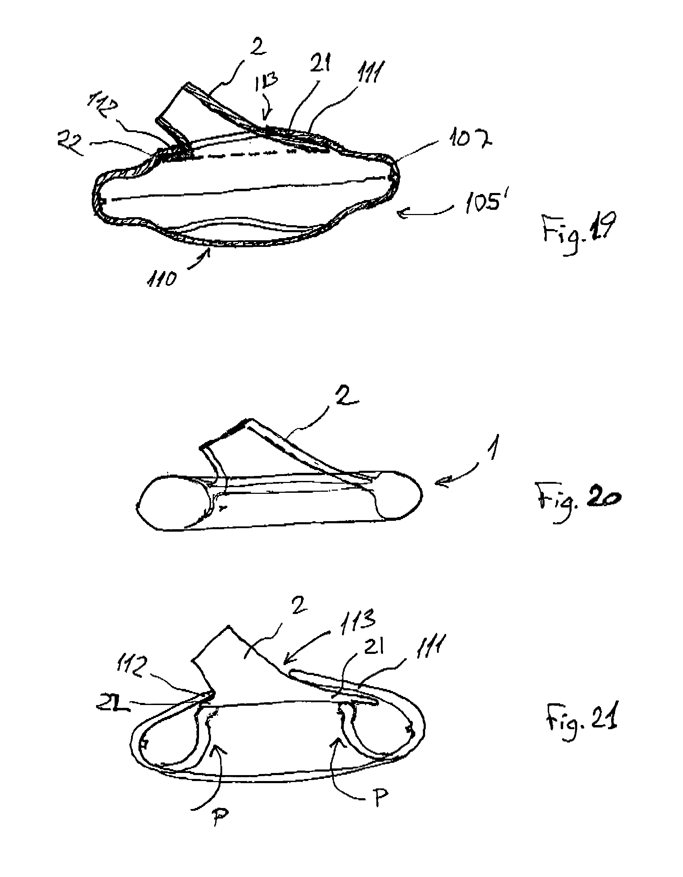

As explained above and with reference to the figures 13 to 21, the cuff 1 is manufactured by blow moulding, where an extruded tubular web 100, for instance made by two strips of material of different thickness, is heated and blown into a balloon 105 inside a cavity 103 in a blow moulding form 101, 102. A strip of material 104 may be provided for reinforcement of the cuff tip. The balloon 105 is subsequently cut off from the tubular piece 100 and removed for further processing.

This further processing includes providing a slit 110 in the middle portion of the balloon 105 and thereby deflating the balloon 105 and subsequently turning the balloon 105 inside out so that inner side becomes the outer side, i.e. into an inverted balloon 105' where any welding fins 107 are located on the inside resulting in a smooth outer surface of the resulting cuff. The advantage by this is that as the burrs or flashes along this assembly line face inside this cannot irritate the tissue in the patient's throat.

After this step of slitting and inverting the balloon 105, the inverted balloon 105' is formed into an annular cuff. In a first embodiment, this is done by stamping out and welding together a central aperture generally corresponding to the footprint of the mask element to which the cuff is to be fixed.

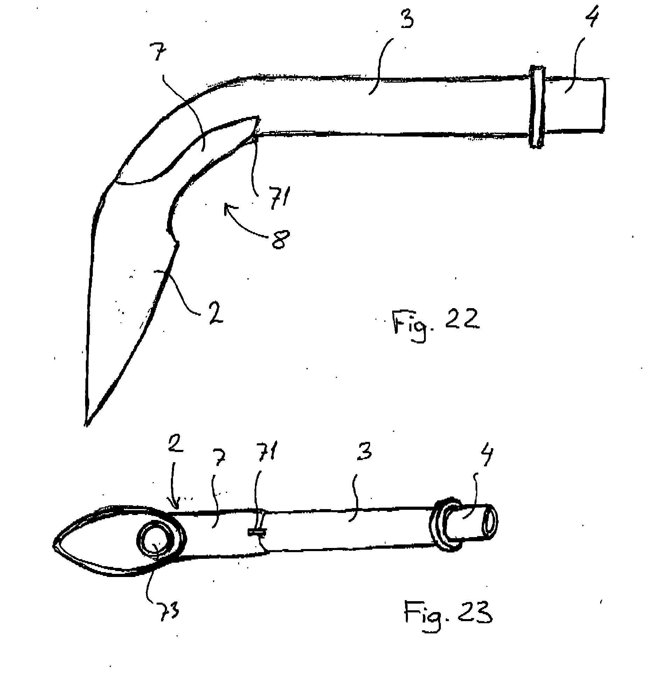

In a second embodiment (see fig. 21), a receiving aperture 113 is provided in the upper side of the balloon 105' for receiving the mask portion 2. The mask portion 2 is provided with mounting surfaces 21 and 22 forming an annular mounting surface around the opening 113 and which makes contact with the annular region 111 and 112 of the inverted balloon 105'. The mask portion 2 is glued to the balloon 105' and is thereby fixed to the inside of the inflatable cuff 1, After this assembly, the cuff is formed by pressing the lower portion of the inverted balloon 105' towards the mask portion as indicated by the arrow P and stamping or otherwise cutting the material and welding the rim edge flange to the mask portion rim flange whereby the inflatable cuff 1 is formed. By this way of assembling the mask portion, a large adhesive contact surface is provided, which in turn results in a firm fixation of the cuff to the mask portion ensuring that the cuff does not disengage during use which would have fatal consequences for the patient. Moreover, by this cuff assembly design the cuff cannot disengage from the mask portion of the laryngeal mask, since even if the adhesive contact fall to function, the cuff will hang from the mask portion since the mask portion is mounted from within.

With reference to figures 22, 23 and 24, a further embodiment of a laryngeal mask according to an aspect of the invention is shown. The laryngeal mask includes a tubular element 3, a mask element 2, where said two elements are joined together by glue or the like. The cuff (not shown in fig. 22) is to be fixed to the mask element 2. A connector piece 4 is provided in the distal end of the tubular element 3 opposite the end 73 of the tubular element 3 where the tubular element 3 is assembled to the mask element 2.

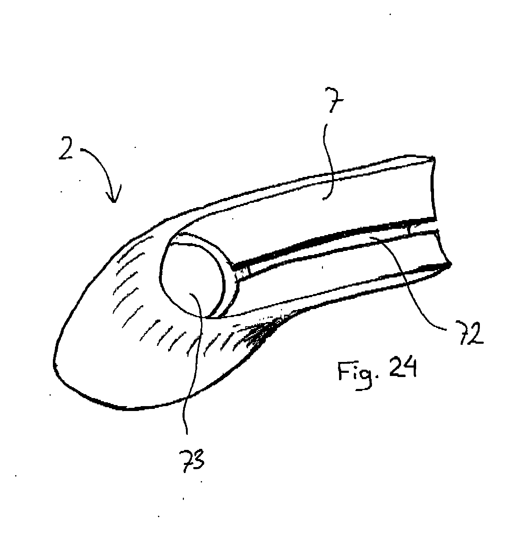

The mask element 2 is adapted to receiving the tubular element 3. On the inside of the bend 8 of the tubular element 3, the mask element 2 is provided with a supporting reinforcement tongue 7 as shown in fig. 1. This tongue 7 supports the tubular element 3 in the bend region and reinforces the laryngeal mask in the area of the bend 8 and prevents the tubular element 3 of the laryngeal mask from kinking, i.e. collapsing during insertion. The tongue 7 is provided with a shape corresponding to the outer contour of the tubular element 3. The inside of this reinforcement tongue 7 is provided with an axially oriented groove 72 (see fig. 24) which corresponds to an axially oriented notch 71 (see figures 22 and 23) provided on the tubular element 3 to facilitate a correct fit between the mask element 2 and the tubular element 3 when said elements are assembled. This ensures a correct assembly of the two elements.

## Claims

1. A laryngeal mask comprising
an airway tube (3),
a mask portion (2),
an inflatable cuff (1),
a connector piece (4),
**characterised in that**
the mask element (2) is provided with a reinforcement tongue (7) for receiving and supporting the tubular element (3) and providing a good adhesive contact between the mask element (2) and the tubular element (3).

2. A laryngeal mask comprising
an inflatable cuff (1),
a mask element (2)
a tubular element (3),
**characterised in that**
the mask element (2) is provided with a reinforcement tongue (7) for receiving and supporting the tubular element (3) and providing an extensive contact for assembly, e.g. by adhesion, between the mask element (2) and the tubular element (3).

3. A laryngeal mask assembly according to claim 1 or 2. wherein the tubular element (3) is provided with a notch (71) which is adapted to cooperate with a corresponding groove (72) provided in the tongue (7) of the mask element (2).

4. A laryngeal mask according to any of claims 1 to 3, wherein there is provided a reinforcement portion in the tubular element (3) by either fitting a tube piece inside the tubular element (3).

5. A laryngeal mask according to any of claims 1 to 4, wherein there is provided a reinforcement portion in the tubular element by prolonging the protruding part of the connector piece (6) further inside the end of the tubular element (3).

6. A laryngeal mask according to any of claims 1 to 5, wherein there is provided a recess in the tube wall of tubular element (3) for the air supply means (8, 9), said recess being provided either internally or externally.

7. A laryngeal mask according to any of claims 1 to 6, wherein there is provided a reinforced cuff tip, preferably by providing a strip of material (104) during the manufacture of the cuff (1).

8. A laryngeal mask according to any of claims 1 to 7, wherein the cuff (1) is provided with a first thickness on a first side and a second thickness on a second side, where said first thickness is larger than the second thickness and that said first side is the side of the cuff (1) facing against the mask element (2).

9. A laryngeal mask according to any of claims 1 to 8, wherein there is provided a bend of approx. 90° above the mask element, and preferably, the wall thickness dimensions in the 2.3-2.8 mm and more preferably with a central portion of 2.7-3.2 mm.

10. A laryngeal mask according to any of the preceding claims, wherein the total wall thickness of the tubular element is preferably 2.2-3.0 mm with the reinforcement tube and 1.2-2.0 mm without reinforcement.

11. A laryngeal mask assembly comprising
an airway tube (3),
a mask portion (2),
an inflatable cuff (1), and
a connector piece (4), wherein at least said airway tube (3), mask portion (2) and inflatable cuff (1) are permanently assembled.

12. A method of manufacture of the cuff including one or more of the following steps:
- extruding a tubular member (100);
- blow moulding a balloon (105) from an extruded tubular member (100);
- slitting the blow moulded balloon (105) and inverting the balloon (105') by turning said balloon (105) inside out;
- punching the central aperture of the cuff and substantially simultaneously welding the inner perimeter of the cuff.

13. A method according to claim 12, including the step of providing e.g. by extruding, a reinforcement strip of material (104) in parallel with the tubular member (100) and joining said strip (104) to the outside of the tubular member (100).

14. A method according to claim 12 or 13, where the tubular member (100) is provided with a first thickness on a first side and a second thickness on a second side, where said first thickness Is larger than the second thickness and that said first side becomes the upper side of the manufactured cuff.

15. A method according to any of claims 12 to 14, wherein a hole is provided in the slit inverted balloon (105') and that the mask portion (2) is adhesively attached to the cuff (1) by positioning the mask portion (2) in said hole.
